# EUROPÄISCHE PATENTSCHRIFT

(11) **EP 3 062 679 B1**
(45) Veröffentlichungstag und Bekanntmachung des Hinweises auf die Patenterteilung: **06.12.2017**
(21) Anmeldenummer: 14790080.7
(22) Anmeldetag: 28.10.2014
(51) Int. Cl.: A61B 1/00, A61B 1/012

(54) **AUSLENKBEWEGUNGSÜBERTRAGUNGSEINRICHTUNG, ENDOSKOPBIEGESTEUERUNG UND ENDOSKOP**
DEVICE FOR TRANSMITTING A DEFLECTION MOVEMENT, ENDOSCOPE BENDING CONTROL UNIT, AND ENDOSCOPE
SYSTÈME DE TRANSMISSION D'UN MOUVEMENT DE DÉVIATION, COMMANDE DE LA COURBURE D'UN ENDOSCOPE ET ENDOSCOPE

(30) Priorität: 30.10.2013 DE 102013222042
(43) Veröffentlichungstag der Anmeldung: 07.09.2016
(73) Patentinhaber: Digital Endoscopy GmbH, 86316 Friedberg (DE)
(72) Erfinder: VIEBACH, Thomas, 86579 Waidhofen (DE); PAUKER, Friedrich, 86420 Diedorf (DE)
(74) Vertreter: TBK
(86) Internationale Anmeldenummer: PCT/EP2014/073066
(87) Internationale Veröffentlichungsnummer: WO 2015/063053

(56) Entgegenhaltungen:
- US-A1- 2006 252 993
- US-A1- 2012 209 068

## Beschreibung

Die vorliegende Erfindung bezieht sich auf eine Auslenkbewegungsübertragungseinrichtung, die eine durch ein Steuerelement bewerkstelligte Auslenkbewegung zu einem Reaktionselement überträgt. Außerdem bezieht sich die vorliegende Erfindung auf eine Endoskopbiegesteuerung und ein Endoskop.

Dokument US 2006/252993 zeigt bereits eine Auslenkbewegungsübertragungseinrichtung mit einem am proximalen Ende der Auslenkbewegungsübertragungseinrichtung angeordnetem Steuerelement zur Bewerkstellung der Auslenkbewegung, einem Basiskörper, an dem das Steuerelement zur Bewerkstellung einer auslenkbewegung so angeordnet ist, dass eine Schwenkbewegung des Steuerelements relativ zum Basiselement ausführbar ist, einem länglichen Übertragungsführungskörper, und einem am distalen Ende der Auslenkbewegungsübertragungseinrichtung angeordneten auszulenkenden biegbaren Körper, wobei das Steuerelement einen Innenkanal aufweist, durch den der auszulenkende biegbare Körper hindurchführbar ist. Solche Auslenkbewegungsübertragungseinrichtungen sind vielseitig anwendbar. Ein Anwendungsgebiet der Auslenkbewegungsübertragungseinrichtung ist ein Endoskop, bei dem ein biegbares Ende eines Katheters, das heißt ein sogenannter Deflectingabschnitt (auch als Biegeabschnitt bezeichnet), durch Schwenken eines Steuerelementes bewegt wird, wobei die Bewegung des Deflectingabschnittes genau der Bewegung des Steuerelementes folgt.
Bei medizinischen Untersuchungen mit einem Endoskop sollte die Übertragung einer Schwenkbewegung eines Steuerelementes zu einer Biegebewegung des Deflectingabschnittes möglichst genau sein. Andererseits sollte die Übertragung einer Schwenkbewegung eines Steuerelementes zu einer Biegebewegung des Deflectingabschnittes für den Anwender einfach und übersichtlich ausführbar sein.

### DURCH DIE ERFINDUNG ZU LÖSENDE AUFGABE

Aufgabe der vorliegenden Erfindung ist es, eine verbesserte Auslenkbewegungsübertragungseinrichtung zu schaffen.
Insbesondere ist es die Aufgabe der vorliegenden Erfindung, eine Auslenkbewegungsübertragungseinrichtung mit besonders günstiger Funktionalität und einfaeher Handhabung zu schaffen. Außerdem sollen eine verbesserte Endoskopbiegesteuerung und ein verbessertes Endoskop geschaffen werden.

### LÖSUNG DER AUFGABE

Diese Aufgabe wird erfindungsgemäß durch eine Auslenkbewegungsübertragungseinrichtung mit den Merkmalen von Anspruch 1 gelöst. Vorteilhafte Weiterbildungen sind Gegenstand der abhängigen Ansprüche. Eine Endoskopbiegesteuerung ist in Anspruch 10 aufgezeigt, und ein Endoskop ist in Anspruch 11 aufgezeigt.

Die Erfindung betrifft somit eine Auslenkbewegungsübertragungseinrichtung mit einem am proximalen Ende der Auslenkbewegungsübertragungseinrichtung angeordneten Steuerelement zur Bewerkstelligung einer Auslenkbewegung, einem länglichen Übertragungsführungskörper, und einem am distalen Ende der Auslenkbewegungsübertragungseinrichtung angeordneten auszulenkenden biegbaren Körper sitzt, wobei das Steuerelement einen Innenkanal aufweist, durch den der auszulenkende biegbare Körper hindurchführbar ist.

In dieser Auslenkbewegungsübertragungseinrichtung ist der auszulenkende biegbare Körper durch das Steuerelement, das die Auslenkbewegung des auszulenkenden biegbaren Körpers steuert, hindurchsteckbar. Somit wird eine Auslenkbewegungsübertragungseinrichtung geschaffen, die auf engstem Raum eine sichere und übersichtliche Handhabung einer Biegebewegung eines Deflectingabschnittes (Biegeabschnitt) ermöglicht.

Die Auslenkbewegungsübertragungseinrichtung kann einen Basiskörper aufweisen, an dem das Steuerelement zur Bewerkstelligung einer Auslenkbewegung so angeordnet ist, dass eine Relativbewegung des Steuerelements zum Basiskörper ausführbar ist, wobei an einer Seite des Basiskörpers der länglichen Übertragungsführungskörper angeordnet ist, und der Basiskörper ebenfalls einen Innenkanal aufweist, durch den der auszulenkende biegbare Körper hindurchführbar ist. Der auszulenkende biegbare Körper ist somit nicht nur durch das Steuerelement sondern durch die gesamte Auslenkbewegungsübertragungseinrichtung hindurchsteckbar.

In der Auslenkbewegungsübertragungseinrichtung kann der Innenkanal konzentrisch im Steuerelement angeordnet sein, und/oder der Innenkanal kann konzentrisch im Basiskörper angeordnet sein. Die Stabilität der gesamten Vorrichtung wird dadurch nicht beeinträchtigt. Darüber hinaus kann der Innenkanal im Steuerelement eine trichterartige Einführöffnung als Einführhilfe aufweisen. Eine sichere Führung des hindurchgesteckten Körpers wird dadurch ermöglicht.

In der Auslenkbewegungsübertragungseinrichtung kann zumindest ein Abschnitt des länglichen Übertragungsführungskörpers durch den Innenkanal des Steuerelements und/oder den Innenkanal des Basiskörpers hindurchführbar sein. Somit kann nicht nur ein Deflectingabschnitt als auszulenkender biegbarer Körper sondern auch ein Übertragungsführungskörper, der die Biegebewegung zum Deflectingabschnitt überträgt, durch die Auslenkbewegungsübertragungseinrichtung hindurchgesteckt werden.

In der Auslenkbewegungsübertragungseinrichtung kann das Steuerelement einen Schwenkabschnitt besitzen, der an einem Kopfabschnitt des Basiskörpers abgestützt ist und zur Bewerkstelligung einer Auslenkbewegung relativ zum Kopfabschnitt des Basiskörpers schwenkbar ist, und zumindest einen Bewegungsübertragungsdrahtkörper besitzen, dessen proximales Ende am Außenumfang des Schwenkabschnittes des Steuerelements angelenkt ist, wobei durch den länglichen Übertragungsführungskörper entlang seiner Längsrichtung der Bewegungsübertragungsdrahtkörper geführt ist, das distale Ende des Bewegungsübertragungsdrahtkörpers am distalen Ende des auszulenkenden biegbaren Körpers angebracht ist, und der Schwenkabschnitt des Steuerelements eine Ausgangsöffnung des Innenkanals des Steuerelement besitzt, und wobei der Kopfabschnitt des Basiskörpers eine Eingangsöffnung des Innenkanals des Basiskörpers besitzt.

In der Auslenkbewegungsübertragungseinrichtung kann das Steuerelement als ein Betätigungshebel aufgebaut sein, der am Schwenkabschnitt eine zum Basiskörper weisende Fußfläche besitzt, wobei der Kopfabschnitt des Basiselements eine zum Steuerelement weisende Stirnfläche besitzt, wobei zumindest die Fußfläche des Schwenkabschnitts des Steuerelements und/oder die Stirnfläche des Kopfabschnittes des Basiselements so gewölbt ist, dass die Fußfläche auf der Stirnfläche abrollbar ist, wobei der Schwenkabschnitt des Steuerelements an seiner Außenseite den Anlenkpunkt des Bewegungsübertragungsdrahtkörpers hat und mit seiner Fußfläche auf der Stirnfläche des Kopfabschnittes des Basiselements schwenkbar sitzt, wobei die Fußfläche des Schwenkabschnitts die Ausgangsöffnung des Innenkanals des Steuerelement aufweist und die Stirnfläche des Kopfabschnittes die Eingangsöffnung des Innenkanals des Basiskörpers aufweist.

In der Auslenkbewegungsübertragungseinrichtung kann das Steuerelement als ein Betätigungshebel aufgebaut sein, der am Schwenkabschnitt in einen Hohlkugelabschnitt übergeht, der zumindest als Kugelringabschnitt ausgebildet ist, wobei der Kopfabschnitt des Basiselements kugelartig ausgebildet ist, wobei der Hohlkugelabschnitt an seiner Außenseite den Anlenkpunkt des Bewegungsübertragungsdrahtkörpers hat und an seiner Innenkugelfläche auf dem kugelartigen Kopfabschnitt gleitfähig sitzt, wobei der Hohlkugelabschnitt die Ausgangsöffnung des Innenkanals des Steuerelement aufweist und der kugelartige Kopfabschnitt die Eingangsöffnung des Innenkanals des Basiskörpers aufweist.

In der Auslenkbewegungsübertragungseinrichtung kann das Steuerelement arretierbar sein, um eine Auslenkstellung des Steuerelements zu arretieren.

In der Auslenkbewegungsübertragungseinrichtung können ein, zwei, drei, vier oder mehr Bewegungsübertragungsdrahtkörper vorgesehen sein, von denen die ersten Enden gleichmäßig zueinander beabstandet am Steuerelement angelenkt sind und die entgegengesetzten zweiten Enden am distalen Endabschnitt des auszulenkenden Körpers in entsprechender Weise gleichmäßig zueinander beabstandet befestigt sind.

Nachstehend ist die Erfindung detailliert anhand von Beispielen erläutert.

### Kurzbeschreibung der Zeichnungen

Figur 1 zeigt eine schematische Schnittansicht der Auslenkbewegungsübertragungseinrichtung eines ersten Ausführungsbeispiels im nicht ausgelenkten Zustand.
Figur 2 zeigt eine schematische Schnittansicht der Auslenkbewegungsübertragungseinrichtung bei einer Auslenkung nach links.
Figur 3 zeigt eine schematische Schnittansicht der Auslenkbewegungsübertragungseinrichtung bei einer Auslenkung nach rechts.
Figur 4 zeigt in einer schematischen perspektivischen ausschnittartigen Darstellung Einzelheiten der Anbindung der Drahtkörper am Steuerelement und wie die Drahtkörper zum Katheterschlauch geführt werden.
Figur 5 zeigt eine schematische Schnittdarstellung der Auslenkbewegungsübertragungseinrichtung des ersten Ausführungsbeispiels, wobei der Katheterschlauch durch die Auslenkbewegungsübertragungseinrichtung geführt ist und nicht ausgelenkt wird.
Figur 6 zeigt eine schematische Schnittdarstellung der Auslenkbewegungsübertragungseinrichtung, wobei der Katheterschlauch durch die Auslenkbewegungsübertragungseinrichtung geführt ist und eine Auslenkung nach links erfolgt.
Figur 7 zeigt eine schematische Schnittdarstellung der Auslenkbewegungsübertragungseinrichtung, wobei der Katheterschlauch durch die Auslenkbewegungsübertragungseinrichtung geführt ist und eine Auslenkung nach rechts erfolgt.
Figur 8 zeigt eine schematische Schnittansicht der Auslenkbewegungsübertragungseinrichtung eines zweiten Ausführungsbeispiels, wobei der Katheterschlauch durch die Auslenkbewegungsübertragungseinrichtung geführt ist und nicht ausgelenkt wird.
Figur 9 zeigt eine schematische Schnittdarstellung der Auslenkbewegungsübertragungseinrichtung des zweiten Ausführungsbeispiels, wobei der Katheterschlauch durch die Auslenkbewegungsübertragungseinrichtung geführt ist und eine Auslenkung nach links erfolgt.
Figur 10 zeigt eine schematische Schnittdarstellung der Auslenkbewegungsübertragungseinrichtung des zweiten Ausführungsbeispiels, wobei der Katheterschlauch durch die Auslenkbewegungsübertragungseinrichtung geführt ist und eine Auslenkung nach rechts erfolgt.

Nachstehend sind Ausführungsbeispiele der vorliegenden Erfindung detailliert anhand der Zeichnungen beschrieben.

### Erstes Ausführungsbeispiel

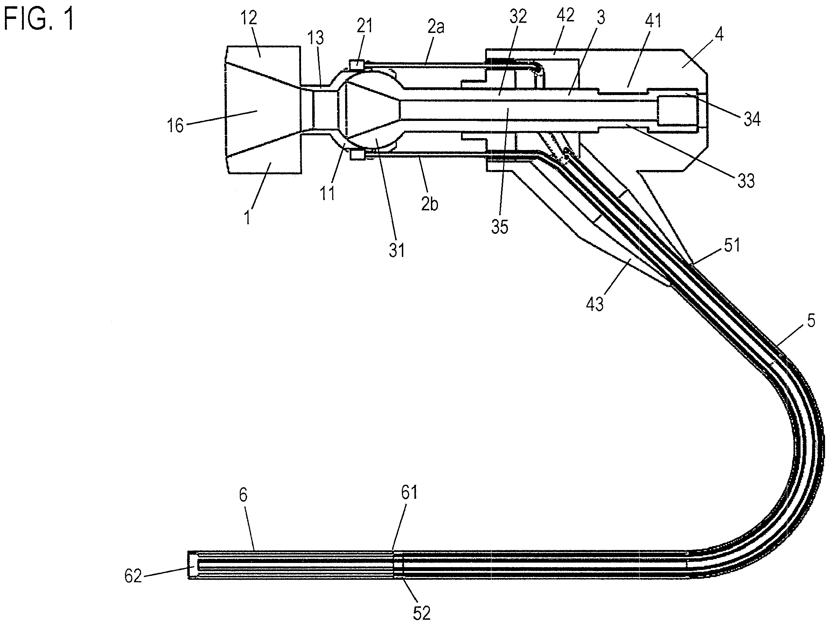

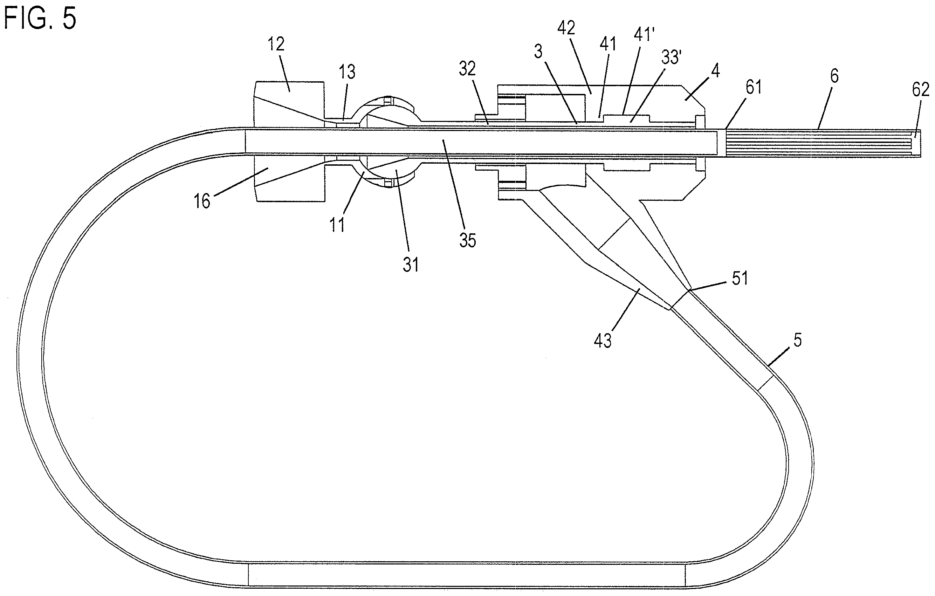

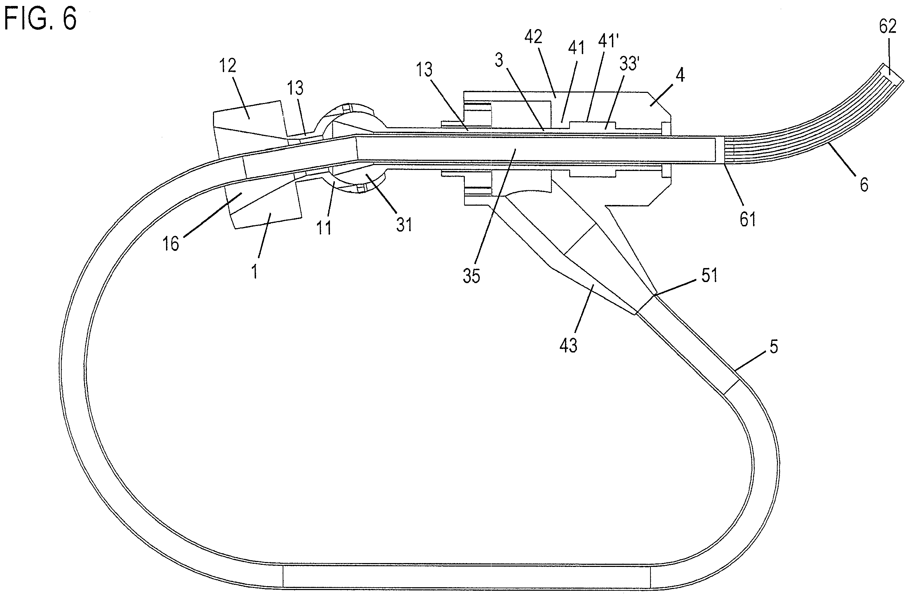

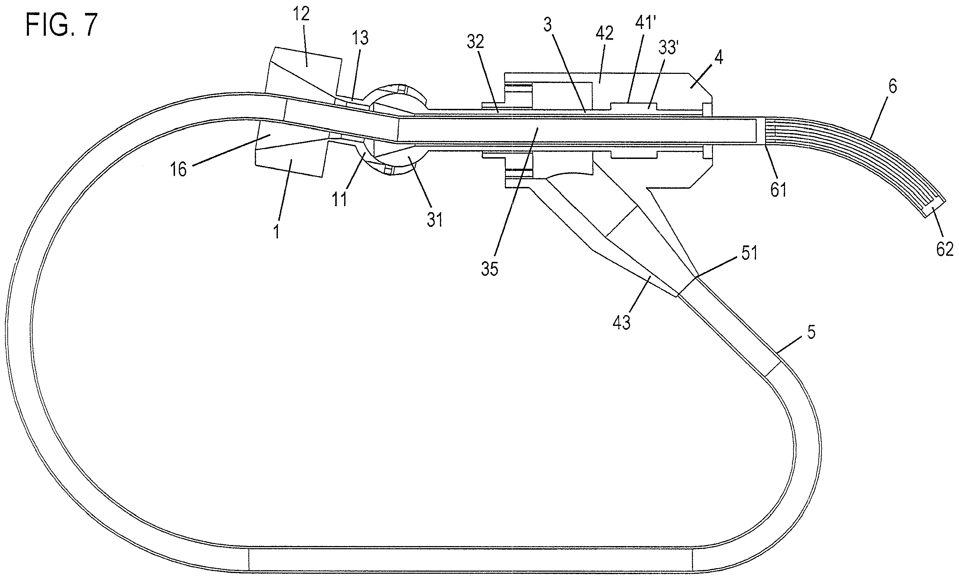

Zunächst ist ein erstes Ausführungsbeispiel der vorliegenden Erfindung detailliert anhand der Figuren 1 - 7 beschrieben.

Das erste Ausführungsbeispiel zeigt ein Auslenkbewegungsübertragungselement, welches bei einem Endoskop für eine Endoskopbiegesteuerung eingesetzt wird.

Diese Auslenkbewegungsübertragungseinrichtung besteht im vorliegenden Ausführungsbeispiel aus einem Steuerelement 1, mehreren Drahtkörpern 2, einem Stabelement 3 als Basiselement, einem Stabelementhalter 4, einem Katheterschlauch 5 und einem biegbaren Körper als Deflectingabschnitt (Biegeabschnitt) 6.

Das Steuerelement 1 besteht aus einem hohlen zylindrischen Element mit einem Steuerkopf 12, an dessen Unterseite ein Hohlstab 13 zentrisch angeordnet ist, der in einen Hohlkugelabschnitt 11 übergeht, an dessen Außenfläche die Drahtkörper 2 verankert sind. Der Hohlkugelabschnitt 11 ist an seiner zum Kopf 12 abgewandten Seite hin offen. Insbesondere ist die Öffnung am Hohlkugelabschnitt 11 derart, dass der Hohlkugelabschnitt 11 zirka 9/10 einer Kugel ausmacht von der zirka 1/10 abgeschnitten ist.

Das Steuerelement 1 ist rotationssymmetrisch aufgebaut und weist einen Innenkanal 16 auf, der sich durch den Kopf 12, den Hohlstab 13 und den Hohlkugelabschnitt 11 konzentrisch erstreckt. Der Innenkanal 16 ist im Kopf 12 derart erweitert, dass sein Innendurchmesser zu der vom Hohlkugelabschnitt 11 abgewandten Seite des Kopfes 12 zunimmt, wie dies in Figur 1 gezeigt ist. Somit hat der Innenkanal 16 des Kopfes 12 eine trichterartige Eingangsöffnung, die in Fig. 1 an der linken Seite des Innenkanals 16 dargestellt ist. Die trichterartige Eingangsöffnung des Innenkanals 16 erleichtert es, den Deflectingabschnitt 6 in den Innenkanal 16 einzuführen.

Das Steuerelement 1 ist aus einem Kunststoffmaterial hergestellt.

Das Steuerelement 1 sitzt als ein Joystick auf einem Kopf 31 des Stabelementes 3. Insbesondere sitzt der Hohlkugelabschnitt 11 des Steuerelementes 1 auf einem Gegenkugelabschnitt 31, der den Kopf des Stabelementes 3 bildet. Der Gegenkugelabschnitt 31 ist dabei derart ausgebildet, dass er eine Kugelform in einer derartigen Größe hat, dass der darauf sitzende Hohlkugelabschnitt 11 leichtgängig bewegbar ist. Die Maßbeziehung zwischen dem Gegenkugelabschnitt 31 und dem Hohlkugelabschnitt 11 sind dabei derart, dass eine Relativbewegung des Steuerelementes 1 zum Stabelement 3 ohne große Kraftanstrengung des Anwenders möglich ist, andererseits aber der Hohlkugelabschnitt 11 nicht lose auf dem Gegenkugelabschnitt 31 sitzt.

Das Stabelement 3 besitzt einen Längszylinder 32, der an seiner distalen Seite in den Gegenkugelabschnitt 31 übergeht und an seinem distalen Endabschnitt ein Schraubende 34 aufweist, das im vorliegenden Ausführungsbeispiel als Innenvierkant ausgearbeitet ist. Distal von dem Vierkantende 34 besitzt das Stabelement 3 an seiner Außenzylinderfläche einen Außengewindeabschnitt 33. Das Stabelement 3 ist rotationssymmetrisch aufgebaut und besitzt in seinem Inneren einen Innenkanal 35, der konzentrisch durch den Gegenkugelabschnitt 31 den Längszylinder 32 und das Vierkantende 34 läuft. Im Übrigen sind der Gegenkugelabschnitt 31, der Längszylinder 32 und das Vierkantende 34 als ein einstückiges Stabelement aufgebaut. Der Längszylinder 32 des Stabelementes 3 ist mit Ausnahme des an ihn vorgesehenen Gewindeabschnittes 33 als ein Zylinder mit einer glatten Außenoberfläche ausgebildet.

Am Gegenkugelabschnitt 31 besitzt der Innenkanal 35 eine trichterartige Eingangsöffnung, die in Fig. 1 an der linken Seite des Innenkanals 35 dargestellt ist. Die trichterartige Eingangsöffnung des Innenkanals 35 steht der Ausgangsöffnung des Innenkanals 16 am Hohlkugelabschnitt 11 gegenüber und erleichtert es, den Deflectingabschnitt 6 in den Innenkanal 35 einzuführen.

Wie dies in den Figuren gezeigt ist, sitzt das Stabelement 3 in einem Stabelementhalter 4. Der Stabelementhalter 4 besteht aus einem Zylinderelement 42, das rotationssymmetrisch aufgebaut ist und einen zentrischen Innenkanal aufweist. Das Zylinderelement 42 weist insbesondere einen zum Steuerelement 1 hinweisenden Hohlraum und einen Boden an der vom Steuerelement 1 abgewandten Seite des Stabelementhalters 4 auf. Genauer gesagt weist der Boden des Stabelementhalters 4 den konzentrischen Innenkanal auf. In dem konzentrischen Innenkanal ist eine Innengewinde 41 ausgearbeitet. Wie dies in den Figuren schematisch angedeutet ist, sitzt das Außengewinde 33 des Stabelementes 3 an dem Innengewinde 41 des Stabelementhalters 4, wobei durch eine Schraubbewegung das Stabelement 3 relativ zum Stabelementhalter 4 konzentrisch hinein oder herausgeschraubt werden kann. Zum Zwecke der Ausführung der Schraubbewegung wird ein entsprechendes Werkzeug in das Vierkantende 34 des Stabelementes 3 eingesetzt. Andere Relativbewegungstechniken sind möglich, wie dies unter "Alternativen" am Ende der Beschreibung dargestellt ist.

An seiner Außenumfangsseite besitzt das Zylinderelement 42 des Stabelementhalters 4 ein Katheteranschlusselement 43. Im vorliegenden Ausführungsbeispiel erstreckt sich das Katheteranschlusselement 43 unter einem spitzen Winkel relativ zu dem Zylinderelement 42 des Stabelementes 4, wie dies aus den Zeichnungen hervorgeht.

Insbesondere ist das Katheteranschlusselement 43 als ein rundes Hohlprofil ausgearbeitet, das quasi eine Kanalabzweigung aus dem distalen Hohlraum des Zylinderelementes 42 darstellt. Das Katheteranschlusselement 43 ist zylinderartig ausgebildet, wobei es sich in Richtung vom Zylinderelement 42 weg verjüngt. Im Inneren besitzt das Katheteranschlusselement 43 einen konzentrischen Kanal, in welchem die Drahtkörper 2 geführt werden. An seinem distalen Ende besitzt das Katheteranschlusselement 43 eine kreisartige Mündung.

An der kreisartigen Mündung des Katheteranschlusselementes 43 ist der Katheterschlauch 5 befestigt. Insbesondere sitzt das proximale Ende 51 des Katheterschlauches 5 an der Mündung des Katheteranschlusselementes 43. An seinem distalen Ende besitzt der Katheterschlauch einen in ihm aufgenommenen Ring 52. Der Ring 52 bildet das distale Ende des Katheterschlauches und den Übergang zu dem Deflectingabschnitt 6.

Der Deflectingabschnitt 6 ist ein biegbarer Körper, der in bekannterweise aus einem elastischem Material hergestellt ist. An seinem proximalen Ende besitzt der Deflectingabschnitt einen Deflectinganschluss 61, an dem er an dem Ring 52 des Katheterschlauches 5 angeschlossen ist. An dem distalen Ende besitzt der Deflectingabschnitt eine Deflectingkappe 62, an der eine Kamera, ein Laser und/oder eine Kamera etc. angeordnet sind. Weitere Funktionseinheiten können an der Deflectingkappe 62 integriert werden.

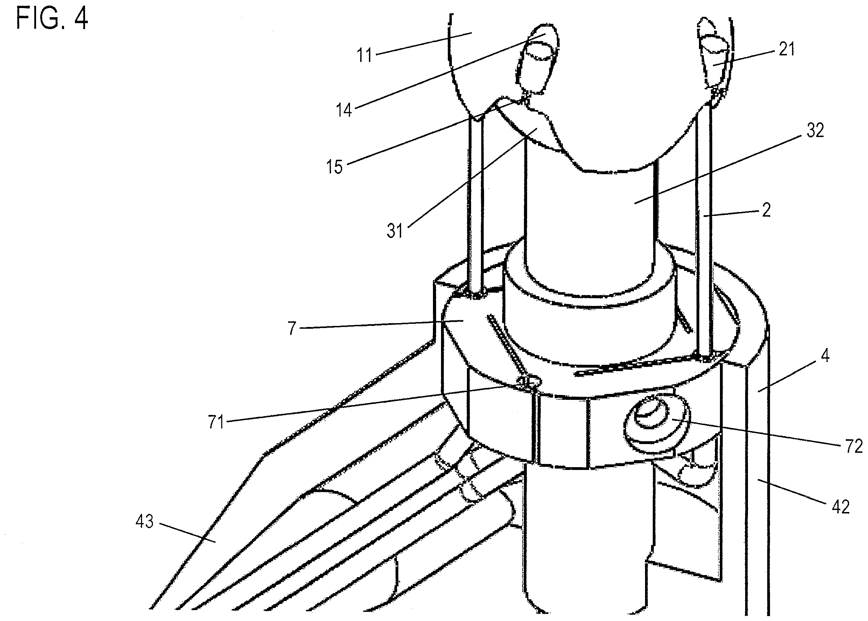

Figur 4 zeigt in einer schematischen perspektivischen ausschnittartigen Darstellung Einzelheiten der Anbindung der Drahtkörper am Steuerelement und wie die Drahtkörper zum Katheterschlauch geführt werden. Der besseren Übersichtlichkeit wegen ist in Fig. 4 der vordere linke Drahtkörper 2 weggelassen worden.

Wie dies in Figur 4 gezeigt ist, sind an der Außenumfangsfläche des Hohlkugelabschnittes 11 an der Äquatorlinie des Hohlkugelabschnittes 11 mehrere Einhängausbauchungen 14 vorgesehen. Im vorliegenden Ausführungsbeispiel sind vier Einhängausbauchungen 14 am Äquator des Hohlkugelabschnittes 11 vorgesehen. Insbesondere sind die Einhängausbauchungen 14 am Hohlkugelabschnitt 11 eingeformte im Querschnitt kreisartige Vertiefungen mit einem Boden, wobei der Boden ungefähr senkrecht zu einer Bohrungsrichtung der Einhängausbauchung 14 verläuft und sich an der Äquatorlinie genauer gesagt senkrecht zum Äquator des Hohlkugelabschnittes 11 befinden. Bei der Herstellung der Einhängausbauchung 14 kann der Hohlkugelabschnitt 11 von der proximalen Seite aus gebohrt werden, sodass die Einhängausbauchung als ein seitlich offenes Sackloch an der Außenumfangsfläche des Hohlkugelabschnittes 11 entsteht. Beliebige andere Herstellverfahren sind hierbei denkbar. Der Außendurchmesser der Einhängausbauchung 14 ist derart gewählt, dass ein Tonnennippel 21 des Drahtkörpers 2 in die Einhängausbauchung 14 hineinpasst. Am Boden der Einhängausbauchung 14, das heißt an dem distalen Ende der Einhängausbauchung 14 ist ein Kanal 15 als Drahtkörpereinhängung ausgearbeitet, der sich koaxial zu der Längserstreckung des Steuerelementes 1 erstreckt und einen Durchmesser hat, der größer als der Außendurchmesser des Drahtkörpers 2 ist, aber kleiner als der Außendurchmesser des Tonnennippels 21 des Drahtes 2 ist. Anders ausgedrückt sind die Einhängausbauchung 14 und die Drahtkörpereinhängung 15 ähnlich wie Baudenzugeinhängungen am Fahrrad so vorgesehen, dass ein Tonnennippel 21 eines Drahtkörpers 2 darin eingehängt werden kann. Das Tonnennippel bildet im eingehängten Zustand des Drahtkörpers 2 das proximale Ende des Drahtkörpers 2.

Im vorliegenden Ausführungsbeispiel sind vier Drahtkörper 2 vorgesehen, von denen in den Figuren 1 bis 3 jeweils zwei Drahtkörper, das heißt der Drahtkörper 2a und der Drahtkörper 2b, dargestellt sind. Die Anzahl an Drahtkörpern 2 ist hierbei nicht begrenzt. Es kann ein Drahtkörper 2, 2, 3, 4 oder mehr Drahtkörper vorgesehen sein. Sollten zwei oder mehr Drahtkörper 2 vorgesehen sein, sind die entsprechenden Einhängausbauchungen 14 gleichmäßig zueinander beabstandet am Äquator des Hohlkugelabschnittes 11 angeordnet.

Wie dies in Figur 4 gezeigt ist, besitzt das Zylinderelement 42 an seinem proximalen Ende das heißt an seinem zum Steuerelement 1 weisenden Ende die Öffnung zum Zylinderelementhohlraum. In diese Öffnung ist ein Drahtkörperführungsring 7 so eingesetzt, dass die proximale Fläche, das heißt die zum Steuerelement 1 weisende Fläche des Drahtführungsringes 7 zu der proximalen, das heißt zum Steuerelement 1 weisenden, Stirnfläche des Zylinderelementes 42 fluchtet. Der Drahtführungsring 7 ist mit tangentialen Schlitzen in gleicher Anzahl, wie Drahtführungen 2 vorhanden sind, versehen, wie dies in Figur 4 gezeigt ist. In den Schlitzen sind Drahtführungsöffnungen 71 gebohrt, die koaxial zu der gemeinsamen Achse des Steuerelementes 1, Stabelementes 4 und des Zylinderelementes 42 des Stabelementhalters 4 verlaufen. Genauer gesagt beträgt der Abstand jeder Einhängausbauchung 14 zu der Mittelachse des Steuerelementes 1 genauso viel wie der radiale Abstand zwischen der Drahtführungsbohrung 71 und der Mittelachse des Drahtführungsringes 7.

Die Drahtkörper 2 werden durch den Katheterschlauch 5 und durch den Ring 52 des Katheterschlauches hindurchgeführt und sind an der Deflectingkappe 62 des Deflectingabschnittes 6 verankert. Insbesondere sind die Drahtkörper 2 so an der Deflectingkappe 62 verankert, dass sie gleichmäßig zueinander beabstandet und in der gleichen Reihenfolge wie am Hohlkugelabschnitt 11 angeordnet, das heißt befestigt sind.

Der Ring 52 weist Öffnungen für die Drahtkörper 2 in entsprechender Weise wie bei der Ausgestaltung des Drahtführungsringes 7 auf.

### Funktionsweise

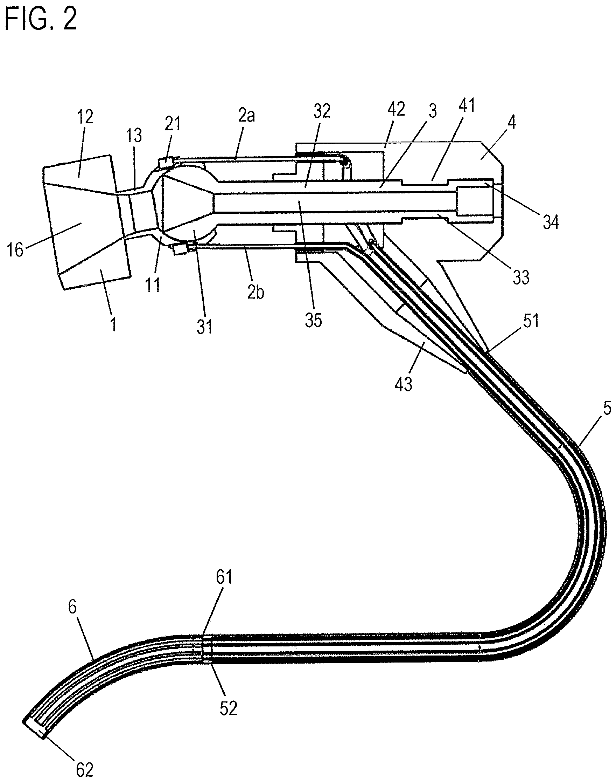

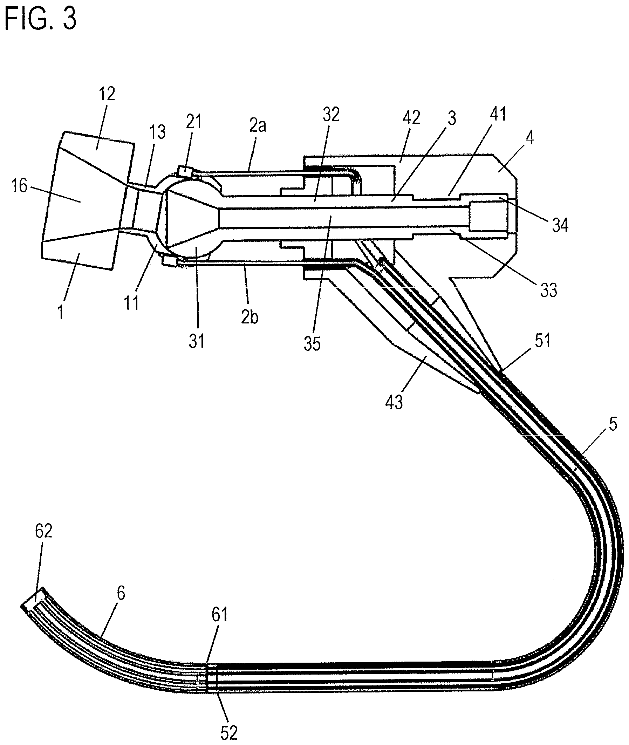

Das Steuerelement 1 kann wie ein Joystick betätigt werden, wobei sein Hohlkugelabschnitt 11 auf dem Gegenkugelabschnitt 31 des Stabelementes 3 bewegt werden kann. Ein Schwenkvorgang des Joysticks 1 relativ zum Stabelement 3 in beliebiger Richtung ist dadurch möglich. Die Richtung und das Ausmaß der Auslenkbewegung des Joysticks 1 relativ zum Stabelement 3 wird dann durch die an der Deflectingkappe 62 angeordneten Drahtkörper 2 auf den als biegbaren Körper ausgestalteten Deflectingabschnitt 6 übertragen. Anders ausgedrückt, wenn der Joystick 1 relativ zum Stabelement 3 nach links bewegt wird, vollführt der Deflectingabschnitt eine nach links gerichtete Bewegung, wie dies in Figur 2 gezeigt ist. Wenn der Joystick 1 relativ zum Stabelement 3 nach rechts bewegt wird, vollführt der Deflectingabschnitt eine nach rechts gerichtete Bewegung, wie dies in Figur 3 der Fall ist.

Das Steuerelement 1 wird vor dem Einführen des Deflectingabschnitts 6 gerade gestellt, so dass der Deflectingabschnitt 6 und der am Deflectingabschnitt 6 angrenzende Abschnitt des Katheterschlauches 5 gerade ausgerichtet sind. Das distale Ende (an der Deflectingkappe 62) des Deflectingabschnitts 6 wird in die trichterartige Eingangsöffnung des Innenkanals 16 im Steuerelement 1 eingeführt, durch den Innenkanal 16 geschoben, in die trichterartige Eingangsöffnung des Innenkanals 35 im Stabelement 3 eingeführt und durch den Innenkanal 35 hindurch geschoben, bis der Deflectingabschnitt 6 an der zu der trichterartigen Eingangsöffnung des Innenkanals 35 entgegengesetzten Ausgangsöffnung des Innenkanals 35 heraustritt.

Wenn der Deflectingabschnitt 6 seine beabsichtigte durch die Auslenkbewegungsübertragungseinrichtung hindurchgeschobene Betriebsposition erreicht hat, kann durch in der erwünschten Richtung und um das erwünschte Maß erfolgendes Schwenken des Steuerelementes 1 der Deflectingabschnitt 6 in die erwünschte Stellung gebracht werden. Das Steuerelement 1 als Joystick kann in alle Richtungen schwenken und somit kann der Deflectingabschnitt 6 nicht nur nach rechts und links, sondern in alle Richtungen schwenken.

### Zweites Ausführungsbeispiel

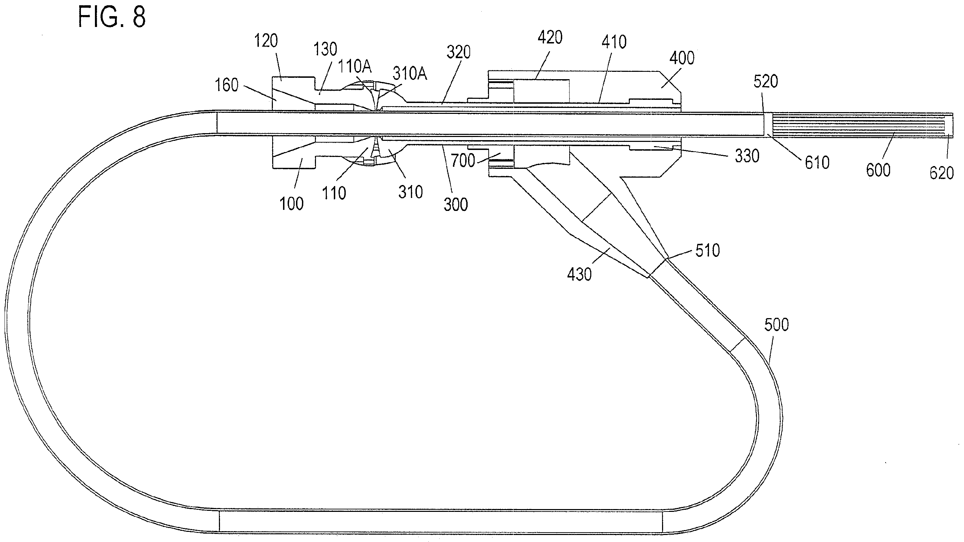

Nachstehend ist ein zweites Ausführungsbeispiel der vorliegenden Erfindung detailliert anhand der Figuren 8 - 10 beschrieben.

Das zweite Ausführungsbeispiel zeigt ein Auslenkbewegungsübertragungselement, welches ebenfalls bei einem Endoskop für eine Endoskopbiegesteuerung eingesetzt wird.

Diese Auslenkbewegungsübertragungseinrichtung besteht auch im vorliegenden Ausführungsbeispiel aus einem Steuerelement 100, mehreren Drahtkörpern (in den Zeichnungen nicht dargestellt) in ähnlicher Weise wie im ersten Ausführungsbeispiel, einem Stabelement 300 als Basiselement, einem Stabelementhalter 400, einem Katheterschlauch 500 und einem biegbaren Körper als Deflectingabschnitt 600.

Das Steuerelement 100 besteht aus einem zylinderartigen Element mit einem Steuerkopf 120, an dessen Unterseite ein Stababschnitt 130 zentrisch angeordnet ist. Der Stababschnitt 130 besitzt an dem zum Steuerkopf 120 entgegengesetzten Ende einen Fußabschnitt 110. Der Stababschnitt 130 hat einen gleichbleibenden Außendurchmesser. Der Fußabschnitt 110 hat einen Außendurchmesser, der in die zum Steuerkopf 120 entgegengesetzte Richtung zunimmt.

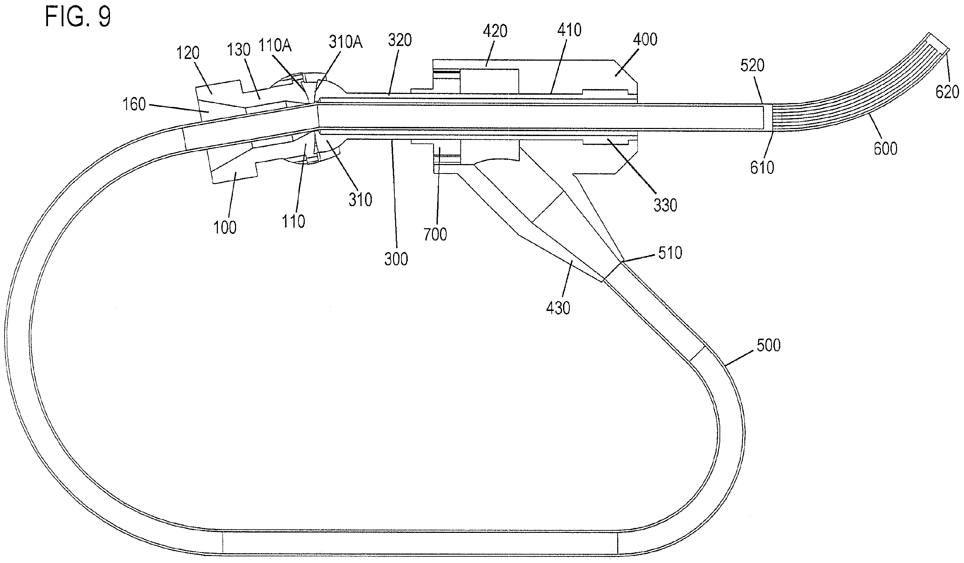

Das Steuerelement 100 ist rotationssymmetrisch aufgebaut und weist einen Innenkanal 160 auf, der sich durch den Kopf 120, den Stababschnitt 130 und den Fußabschnitt 110 konzentrisch erstreckt. Der Innenkanal 160 ist im Kopf 120 derart erweitert, dass sein Innendurchmesser zu zum Fußabschnitt 110 hin weisenden Seite zunimmt, wie dies in Figur 9 gezeigt ist. Somit hat der Innenkanal 160 des Kopfes 120 eine trichterartige Eingangsöffnung, die in Fig. 9 an der linken Seite des Innenkanals 160 dargestellt ist. Die trichterartige Eingangsöffnung des Innenkanals 160 erleichtert es, den Deflectingabschnitt 600 in den Innenkanal 160 einzuführen. Die trichterartige Eingangsöffnung des Innenkanals 160 ist abgerundet, so dass sie keine scharfen Kanten aufweist.

Am Fußabschnitt 110 schwenkt das Steuerelement 100 relativ zum Stabelement 300; daher ist der Fußabschnitt 110 des Steuerelements 100 als Schwenkabschnitt 110 bezeichnet.

Der Schwenkabschnitt 110 besitzt an der zum Steuerkopf 120 entgegengesetzten Seite eine als Fußfläche 110A ausgebildete Endfläche. Im vorliegenden Ausführungsbeispiel ist die Fußfläche 110A nach außen gewölbt, d.h. entgegengesetzt zur die trichterartige Eingangsöffnung des Innenkanals 160 aufweisenden Stirnfläche des Steuerkopfes 120 weg nach außen gewölbt. Anders ausgedrückt nimmt der in Längsrichtung des zylindrischen Steuerelements 100 gemessene Abstand zwischen der Fußfläche 110A zu der zum Fuß entgegengesetzten Stirnfläche des Steuerkopfes 120 vom Außenumfang zur Mitte hin zu. Die Fußfläche 110A bildet daher einen Abschnitt einer Kugelfläche mit einem vorbestimmten Radius, dessen Mittelpunkt auf der gedachten verlängerten Achse des Steuerelements 100 liegt.

An der Fußfläche 110A besitzt der Innenkanal 160 seine Ausgangsöffnung. Der Innenkanal 160 verjüngt sich in dem Bereich des Fußabschnittes 110 und erreicht an der Fußfläche 110A seinen geringsten Innendurchmesser. An der Ausgangsöffnung ist der Innenkanal 160 abgerundet, so dass er keine scharfen Kanten aufweist.

Der Innenkanal 160 hat an dieser engsten Stelle einen Innendurchmesser, der einerseits noch ein sicheres Hindurchgleiten des Deflectingabschnittes 600 und des Katheterschlauches 500 ermöglicht und andererseits eine Führung für den Deflectingabschnitt 600 und den Katheterschlauch 500 bildet, wenn diese hindurchgesteckt sind.

Das Steuerelement 100 ist rotationssymmetrisch, wie dies in den Figuren 9 - 11 erkennbar ist. Das Steuerelement 100 ist aus einem Kunststoffmaterial hergestellt, kann aber auch aus Metall gefertigt sein.

Der Fußfläche 110A des Schwenkabschnittes 110 steht eine Stirnfläche 310A eines Kopfabschnittes 310 des Stabelementes 300 gegenüber, wie dies in den Figuren 8 - 10 dargestellt ist.

Das Stabelement 300 hat einen Längszylinder 320, der an seiner distalen Seite in den Kopfabschnitt 310 übergeht und in der Nähe seines distalen Endabschnitts eine Durchmessererweiterung 330 aufweist. Die Durchmessererweiterung 330 ist als ein Zylinderabschnitt vorgesehen, der einen größeren Außendurchmesser als in dem Abschnitt zwischen dem Kopfabschnitt 310 und der Durchmessererweiterung 330 besitzt. Das Stabelement 300 ist rotationssymmetrisch aufgebaut.

Das Stabelement 300 ist somit ein einstückiges längliches Element, das den Kopfabschnitt 310, den Längszylinder 320 und die Durchmessererweiterung 330 aufweist. Der Längszylinder 320 des Stabelementes 300 mit einer glatten Außenoberfläche ausgebildet.

Das Stabelement 300 ist rotationssymmetrisch aufgebaut und weist einen Innenkanal 350 auf, der sich durch den Kopfabschnitt 310, den Längszylinder 320 und die Durchmessererweiterung 330 konzentrisch erstreckt. Der Innenkanal 350 ist am Kopfabschnitt 310 verengt. Dabei besitzt der Innenkanal 350 an der Stirnfläche 310A des Kopfabschnittes 310 seine Eingangsöffnung, an der der Innenkanal 350 seinen geringsten Innendurchmesser hat. An der Eingangsöffnung ist der Innenkanal 350 abgerundet, so dass er keine scharfen Kanten aufweist. Stromabwärtig der Eingangsöffnung besitzt der Innenkanal 350 einen gleichmäßigen Innendurchmesser, der größer als am Kopfabschnitt 310 ist. Auch an der Ausgangsöffnung ist der Innenkanal 350 abgerundet, so dass er keine scharfen Kanten aufweist. Die Verengung des Innendurchmessers am Kopfabschnitt 310 ermöglicht einerseits noch ein sicheres Hindurchgleiten des Deflectingabschnittes 600 und des Katheterschlauches 500 und bildet andererseits eine Führung für den Deflectingabschnitt 600 und den Katheterschlauch 500, wenn diese hindurchgesteckt sind.

Im vorliegenden Ausführungsbeispiel ist die Stirnfläche 310A ebenfalls nach außen zum Steuerelement 100 hin gewölbt. Anders ausgedrückt erhebt sich die Fußfläche 110A vom Außenumfang zur Mitte hin in Richtung zum Steuerelement 100. Die Stirnfläche 310A bildet dabei einen Abschnitt einer Kugelfläche mit einem vorbestimmten Radius, dessen Mittelpunkt auf der gedachten verlängerten Achse des Stabelements 300 liegt.

Die Fußfläche 110A und die Stirnfläche 310A stehen sich gegenüber und befinden sich in Kontakt zueinander. Somit sitzt der Schwenkabschnitt 110 des Steuerelements 100 mit seiner Fußfläche 110A schwenkbar auf der Stirnfläche 310A des Kopfabschnittes 310 des Stabelements 300. Anders ausgedrückt, kann die Fußfläche 110A auf der Stirnfläche 310A abrollen.

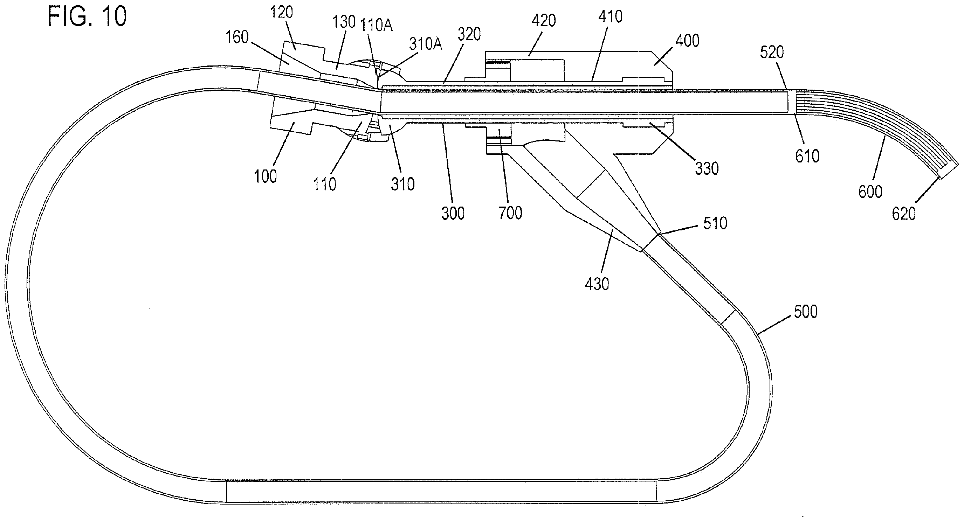

Im nicht geschwenkten Zustand liegen der Schwenkabschnitt 110 des Steuerelements 100 und der Kopfabschnitt 310 des Stabelements 300 auf der gleichen Mittelachse, da im nicht geschwenkten Zustand das Steuerelement 100 und das Stabelement 300 koaxial zueinander angeordnet sind. Somit berühren sich im nicht geschwenkten Zustand die Fußfläche 110A und die Stirnfläche 310A an einer Kreislinie, wie dies in Figur 9 gezeigt ist. Wenn das Steuerelement 100 geschwenkt wird, wie dies in den Figuren 10 und 11 gezeigt ist, also der Schwenkabschnitt 110 relativ zum Kopfabschnitt 310 des Stabelements 300 geneigt wird, rollt die Fußfläche 110A auf der Stirnfläche 310A. Es wird hierbei darauf hingewiesen, dass die Darstellung in den Zeichnungen lediglich schematisch ist.

Das Steuerelement 100 sitzt somit als ein Joystick auf dem Kopfabschnitt 310 des Stabelementes 300.

Das Stabelement 300 ist in dem Stabelementhalter 400 angeordnet. Der Stabelementhalter 400 ist als ein Zylinderelement 420 gebildet, das rotationssymmetrisch aufgebaut ist. Das Zylinderelement 420 weist an der zum Steuerelement 100 hin weisenden Seite einen Hohlraum und einen Boden an der vom Steuerelement 100 abgewandten Seite des Stabelementhalters 400 auf. Der Boden des Stabelementhalters 400 weist einen konzentrischen Innenkanal 410 auf. In dem konzentrischen Innenkanal 410 ist an einem Abschnitt von diesem ein Hohlraum ausgearbeitet, in welchem die Durchmessererweiterung 330 des Stabelementes 300 sitzt. Die axialen Endflächen des Hohlraums in der Nähe des Bodens des Stabelementhalters 400 bilden jeweilige Anschläge für die axialen Endflächen der Durchmessererweiterung 330. In diesem Ausführungsbeispiel kann sich somit das Stabelement 300 ist axial relativ zum Stabelementhalter 400 nicht oder lediglich mit einem geringfügigen Spiel bewegen.

An einem Abschnitt seiner Außenumfangsseite besitzt das Zylinderelement 420 des Stabelementhalters 400 ein Katheteranschlusselement 430. Im vorliegenden Ausführungsbeispiel erstreckt sich das Katheteranschlusselement 430 unter einem spitzen Winkel relativ zu dem Zylinderelement 420 des Stabelementes 400, wie dies aus den Zeichnungen hervorgeht.

Insbesondere ist das Katheteranschlusselement 430 als ein rundes Hohlprofil ausgearbeitet, das quasi eine Kanalabzweigung aus dem distalen Hohlraum des Zylinderelementes 420 darstellt. Das Katheteranschlusselement 430 ist zylinderartig ausgebildet, wobei es sich in Richtung vom Zylinderelement 420 weg verjüngt. Im Inneren besitzt das Katheteranschlusselement 430 einen konzentrischen Kanal, in welchem die Drahtkörper geführt werden. An seinem distalen Ende besitzt das Katheteranschlusselement 430 eine kreisartige Mündung.

An der kreisartigen Mündung des Katheteranschlusselementes 430 ist der Katheterschlauch 500 befestigt. Insbesondere sitzt das proximale Ende 510 des Katheterschlauches 500 an der Mündung des Katheteranschlusselementes 430.

An seinem distalen Ende besitzt der Katheterschlauch einen in ihm aufgenommenen Ring 520. Der Ring 520 bildet das distale Ende des Katheterschlauches und den Übergang zu dem Deflectingabschnitt 600.

Der Katheterschlauch 500 und der Deflectingabschnitt 600 sind ähnlich aufgebaut wie der Katheterschlauch 5 und der Deflectingabschnitt 6 des ersten Ausführungsbeispiels.

Ähnlich wie in Fig. 4 sind auch im zweiten Ausführungsbeispiel die Drahtelemente an Einhängausbauchungen des Steuerelements eingehängt. Die Beschreibung in Zusammenhang mit den Drahtelementen des ersten Ausführungsbeispiels gilt auch im zweiten Ausführungsbeispiel und wird nicht wiederholt. Der Aufbau, die Führung und Funktionsweise der Drahtelemente ist gleich.

Lediglich der Drahtführungsring 700 hat im zweiten Ausführungsbeispiel zusätzlich zu seinem im ersten Ausführungsbeispiel beschriebenen Aufbau eine weitere Funktion. Der Drahtführungsring 700 als solcher ist wie im ersten Ausführungsbeispiel aufgebaut. Der Drahtführungsring 700 des zweiten Ausführungsbeispiels ist längs zum Zylinderelement 420 des Stabelementhalters 400 verschiebbar. Zu diesem Zweck weist das Zylinderelement 420 des Stabelementhalters 400 einen in den Zeichnungen nicht gezeigten axialen Längsschlitz auf, entlang welcher die Befestigungsschraube, die in die Gewindebohrung 72 (Figur 4) eingreift und den Drahtführungsring 700 am Zylinderelement 420 des Stabelementhalters 400 fixieren kann, an verschiedenen Positionen festgeschraubt werden kann.

Die Drähte können hierbei gespannt werden, indem das Ringelement 700 in axialer Richtung in den Hohlraum des Zylinderelements 420 des Stabelementhalters 400 hinein verschoben und dann per Feststellelement arretiert wird. Dadurch ändert sich der Winkel des Ablenkungspunktes der Drähte am Ausgang des Ringelementes 700; das heißt an der rechten Seite des Ringelementes 700 in Fig. 8. Anders ausgedrückt wird beim Spannen der Drähte der stumpfe Winkel des Ablenkungspunktes der Drähte am stromabwärtigen Ausgang des Ringelementes 700 zwischen der Erstreckung der Drähte vom Einhängpunkt zum Ringelement 700 und der Erstreckung der Drähte vom Ringelement 700 zum Katheterschlauch 500 verkleinert. Dadurch können kleine Spannlängen der Drähte verwirklicht werden.

Die Drahtkörper sind durch den Katheterschlauch 500 und durch den Ring 520 des Katheterschlauches hindurchgeführt und sind an der Deflectingkappe 620 des Deflectingabschnittes 600 verankert. Insbesondere sind die Drahtkörper so an der Deflectingkappe 620 verankert, dass sie gleichmäßig zueinander beabstandet und in der gleichen Reihenfolge wie am Schwenkabschnitt 110 angeordnet sind.

Der Ring 520 weist Öffnungen für die Drahtkörper in entsprechender Weise wie bei der Ausgestaltung des Drahtführungsringes 700 auf.

Die Länge jedes Drahtkörpers vom Befestigungspunkt an der Deflectingkappe 620 bis zum Befestigungspunkt am Schwenkabschnitt 110 ist stets gleich.

### Funktionsweise

Ähnlich wie im ersten Ausführungsbeispiel kann auch das Steuerelement 100 des zweiten Ausführungsbeispiels wie ein Joystick betätigt werden, wobei die Fußfläche 110A des Hohlkugelabschnitts 110 auf der Stirnfläche 310A des Kopfabschnittes 310 abrollt. Ein Schwenkvorgang des Joysticks 100 relativ zum Stabelement 300 in beliebiger Richtung ist dadurch möglich. Die Richtung und das Ausmaß der Auslenkbewegung des Joysticks 100 relativ zum Stabelement 300 wird dann durch die an der Deflectingkappe 620 angeordneten Drahtkörper auf den als biegbaren Körper ausgestalteten Deflectingabschnitt 600 übertragen.

Wenn der Joystick 100 relativ zum Stabelement 300 nach links bewegt wird, vollführt der Deflectingabschnitt 600 eine nach links gerichtete Bewegung, wie dies in Figur 9 gezeigt ist. Wenn der Joystick 100 relativ zum Stabelement 300 nach rechts bewegt wird, vollführt der Deflectingabschnitt 600 eine nach rechts gerichtete Bewegung, wie dies in Figur 10 gezeigt ist.

Das Steuerelement 100 wird vor dem Einführen des Deflectingabschnitts 600 gerade gestellt, so dass der Deflectingabschnitt 600 und der am Deflectingabschnitt 600 angrenzende Abschnitt des Katheterschlauches 500 gerade ausgerichtet sind. Das distale Ende (an der Deflectingkappe 620) des Deflectingabschnitts 600 wird in die trichterartige Eingangsöffnung des Innenkanals 160 im Steuerelement 100 eingeführt, durch den Innenkanal 160 geschoben, in die zur Ausgangsöffnung des Innenkanals 160 benachbarten Eingangsöffnung des Innenkanals 350 im Stabelement 300 eingeführt und durch den Innenkanal 350 hindurch geschoben, bis der Deflectingabschnitt 600 an der zu der trichterartigen Eingangsöffnung des Innenkanals 350 entgegengesetzten Ausgangsöffnung des Innenkanals 350 heraustritt.

Wenn der Deflectingabschnitt 600 seine beabsichtigte durch die Auslenkbewegungsübertragungseinrichtung hindurchgeschobene Betriebsstellung erreicht hat, kann durch in der erwünschten Richtung und um das erwünschte Maß erfolgendes Schwenken des Steuerelementes 100 der Deflectingabschnitt 600 in die erwünschte Stellung gebracht werden.

### Alternativen

Der Drahtkörper 2 ist in der in Figur 4 gezeigten Einhängausbauchung 14 in der Form eines Tonnennippels 21 eingehängt. Die Erfindung ist nicht auf ein Tonnennippel beschränkt und das Nippel 21 kann als ein bekanntes Birnennippel ausgeführt sein, beliebige ähnliche Nippel können angewendet werden. Die Form der Einhängausbauchung 14 kann an die gewählte Nippelform angepasst werden.

Im ersten Ausführungsbeispiel ist die Größe des Hohlkugelabschnittes 11 so gewählt worden, dass sie 9/10 einer Kugel beträgt. Die Erfindung ist nicht darauf beschränkt. Eine beliebige Hohlkugelformgröße des Hohlkugelabschnittes 11 kann gewählt werden, solange diese noch an dem Gegenkugelabschnitt 31 die Schwenkbewegung ausführen kann. Der Hohlkugelabschnitt 11 kann auch eine Hohlkugelringabschnittform haben, die sich um ein vorbestimmtes Mindestmaß parallel zur Achsrichtung des Steuerelements 1 zu beiden Seiten von der Äquatorlinie erstreckt und quasi ein Äquatorband bildet.

Im zweiten Ausführungsbeispiel ist die Fußfläche 110A nach außen gewölbt. Außerdem ist die Stirnfläche 310A des Kopfabschnittes 310 nach außen gewölbt. Die Erfindung ist nicht darauf beschränkt. Die Auslenkbewegungsübertragungseinrichtung kann unter Ausnutzung des Prinzips der Erfindung auch so aufgebaut sein, dass die Fußfläche 110A eben gestaltet ist und die Stirnfläche 310A nach außen gewölbt ist. Andererseits kann die Auslenkbewegungsübertragungseinrichtung auch so aufgebaut sein, dass die Fußfläche 110A nach außen gewölbt ist und die Stirnfläche 310A eben gestaltet ist. Es ist auch eine Konstruktion denkbar, bei der die Stirnfläche 310A nach innen gewölbt ist und die Fußfläche 110A nach außen gewölbt ist, sofern der Krümmungsradius der Stirnfläche 310A größer als der Krümmungsradius der Fußfläche 110A ist. In ähnlicher Weise kann die Stirnfläche 310A nach außen gewölbt sein und die Fußfläche 110A nach innen gewölbt sein, sofern der Krümmungsradius der Stirnfläche 310A kleiner als der Krümmungsradius der Fußfläche 110A ist. Es ist lediglich ausreichend, dass die Fußfläche 110A sicher und kontrolliert auf der Stirnfläche 310A abrollen kann.

Im ersten Ausführungsbeispiel dient das Vierkantende 34 der Ermöglichung einer Schraubbewegung, um an den Gewindeabschnitten 33 und 41 eine Relativbewegung des Stabelementes 3 zum Stabelementhalter 4 auszuführen, um die Drahtkörper 2 zu spannen. Die Erfindung ist nicht auf die Vierkantform am Ende 34 des Stabelementes 3 beschränkt. Eine Dreikantform, Achtkantform, andere polygonale Form kann gewählt werden. Im Prinzip kann jede beliebige Form, die das Angreifen eines die Drehbewegung des Stabelementes am Ende 34 erzeugenden Momentes ermöglicht, gewählt werden.

Im ersten Ausführungsbeispiel wird die Relativbewegung des Stabelementes 3 zum Stabelementhalter 4 durch die Gewindeabschnitte 33 und 41 bewerkstelligt. Durch die Bewegung des Stabelementes 3 relativ zum Stabelementhalter 4 werden die Drahtkörper 2 gespannt. Eine beliebige andere Bewegungsart des Stabelementes 3 zum Stabelementhalter 4 kann für diesen Zweck gewählt werden. Beispielsweise kann der Stabelementhalter 4 ein durchgehendes Innenzylinderloch aufweisen und das Stabelement 3 einen durchgehend zylindrischen Längszylinder 32 haben, wobei am Ende 34 des Stabelementes 3 eine Zugeinrichtung angebracht ist. Im Stabelementhalter 4 kann eine Gewindebohrung senkrecht zur Achse des Stabelementhalters vorgesehen werden, in der eine Feststellschraube sitzt, die den Längszylinder 32 in beliebiger Stellung relativ zum Stabelementhalter 4 arretieren kann.

Im ersten Ausführungsbeispiel ist das Stabelement 3 relativ zum Stabelementhalter 4 durch eine Schraubbewegung konzentrisch hinein oder herausschraubbar, wobei bei der Schraubbewegung das Innengewinde 41 des Stabelementhalters 4 am Außengewinde 33 des Stabelementes 3 in Gewindeeingriff stehen. Bei dieser Ausführung sind die Drähte 2 spannbar, indem das Stabelement 3 relativ zum Stabelementhalter 4 bewegt und arretiert wird.

Im zweiten Ausführungsbeispiel sitzt das Stabelement 300 im Stabelementhalter 400, in dem die Durchmessererweiterung 330 in einem für diese vorgesehenen Hohlraum des Stabelementhalters 400 angeordnet ist. Die distale und proximale Endfläche der Durchmessererweiterung 330 bildet dabei jeweils einen Anschlag am Hohlraum des Stabelementhalters 400. Bei dieser Ausführung sind die Drähte spannbar, indem das Ringelement 700 in axialer Richtung in den Hohlraum des Zylinderelements 420 des Stabelementhalters 400 hinein verschoben und dann per Feststellelement arretiert wird.

Die Erfindung ist nicht darauf beschränkt. Im ersten Ausführungsbeispiel kann die für das zweite Ausführungsbeispiel vorgesehene Drahtspannmöglichkeit angewendet werden; und im zweiten Ausführungsbeispiel kann die für das erste Ausführungsbeispiel vorgesehene Drahtspannmöglichkeit angewendet werden.

In den Ausführungsbeispielen erstreckt sich das Katheteranschlusselement 43; 430 unter einem spitzen Winkel unter Betrachtung der Figuren zum Stabelementhalter 4; 400. Die Erfindung ist nicht darauf beschränkt. Ein beliebiger Erstreckungswinkel des Katheteranschlusselementes zum Stabelementhalter kann gewählt werden.

In den Ausführungsbeispielen ist der Katheterschlauch 5; 500 ein Übertragungsführungskörper, der einen Hohlraum besitzt, in dem der Bewegungsübertragungsdrahtkörper geführt wird. Bei einer Schwenkbewegung des Steuerelementes sind die Bewegungsübertragungsdrahtkörper 2 Zugkräften und Druckkräften ausgesetzt. Wenn diese Zugkräfte und Druckkräfte auf sie ausgeübt werden, müssen die Bewegungsübertragungsdrahtkörper 2 am Übertragungsführungskörper gleiten können. Der Übertragungsführungskörper kann hierbei einen geschlossenen Querschnitt haben, wie dies bei einem Katheterschlauch 5; 500 der Fall ist. Die Erfindung ist nicht darauf beschränkt. Der Übertragungsführungskörper kann ein Schienenelement oder Kastenelement sein, an dem der Bewegungsübertragungsdrahtkörper geführt wird. Der Querschnitt des Übertragungsführungskörpers kann an der Seite offen sein, an der die Bewegungsübertragungsdrahtkörper 2 nicht gleiten.

Das Steuerelement 1; 100 kann arretierbar sein, um eine Auslenkstellung des Steuerelements 1; 100 zu arretieren. Im ersten Ausführungsbeispiel kann die Arretierung durch eine Feststellschraube erfolgen, die z.B. den Hohlkugelabschnitt 11 durchdringt und an der Oberfläche des Gegenkugelabschnittes 31 angreift und somit als eine Reibungsbremse so wirkt, dass eine bestimmte Auslenkstellung des Steuerelements 1 also des Steuerhebels per Reibungsbremse arretierbar ist. Darüber hinaus kann in allen Ausführungsbeispielen eine Arretierung erfolgen, indem der/die Bewegungsübertragungsdrahtkörper 2 z.B. am Drahtführungsring 7; 700 oder am Zylinderelement 42; 420 des Stabelementhalters 4; 400 geklemmt werden. Sollten alle Drähte 2 z.B. durch eine am Drahtführungsring 7; 700 oder am Zylinderelement 42; 420 angebrachte Feststellklemme arretiert werden, wird dadurch eine sichere Arretierung einer Auslenkstellung des Steuerelements 1; 100 erreicht. Andere technische Möglichkeiten zum Blockieren der Drähte 2 können gewählt werden.

In den Ausführungsbeispielen ist die Auslenkbewegungsübertragungseinrichtung auf eine Endoskopbiegesteuerung bei einem Endoskop angewandt. Die Auslenkbewegungsübertragungseinrichtung kann auch auf anderen technischen Gebieten Anwendung finden. Ein Einsatz in Wasser führenden Kanälen, in Bergbaustollen etc. ist denkbar. Die Erfindung kann überall dort, wo Schwenkbewegungen in Auslenkbewegungen eines Auslenkelementes umgewandelt werden, angewandt werden.

### Bezugszeichenliste

1 Steuerelement; Joystick
2, 2a, 2b Drahtkörper
3 Basiselement; Stabelement
4 Stabelementhalter
5 Katheterschlauch
6 biegbarer Körper, Deflectingabschnitt
7 Drahtführungsring
11 Hohlkugelabschnitt
11A Fußfläche des Hohlkugelabschnitts 11
12 Kopf des Steuerelementes 1
13 Stababschnitt
14 Einhängausbauchung
15 Drahtkörpereinhängung
16 Innenkanal im Steuerelement
21 Tonnennippel
31 Kopfabschnitt; Gegenkugelabschnitt
31A Stirnfläche des Kopfabschnittes 31
32 Längszylinder
33 Gewindeabschnitt des Stabelementes 3
34 Vierkantende; distales Ende des Stabelementes 3
35 Innenkanal im Stabelement
41 Gewindeabschnitt des Stabelementhalters 4
42 Zylinderelement
43 Katheteranschlusselement
51 Katheterschlauchanschluss
52 Ring
61 Deflectinganschluss
62 Deflectingkappe
71 Drahtführungsbohrung
72 Gewindebohrung für Befestigungsschraube
100 Steuerelement; Joystick
110 Hohlkugelabschnitt
110A Fußfläche des Hohlkugelabschnitts 110
120 Kopf des Steuerelementes 100
130 Stababschnitt
160 Innenkanal im Steuerelement
300 Basiselement; Stabelement
310 Kopfabschnitt; Gegenkugelabschnitt
310A Stirnfläche des Kopfabschnittes 31
320 Längszylinder
330 Durchmessererweiterung
350 Innenkanal im Stabelement
400 Stabelementhalter
410 Innenkanal im Stabelement
420 Zylinderelement
430 Katheteranschlusselement
500 Katheterschlauch
510 Katheterschlauchanschluss
520 Ring
600 biegbarer Körper, Deflectingabschnitt
610 Deflectinganschluss
620 Deflectingkappe
700 Drahtführungsring

## Patentansprüche

1. Auslenkbewegungsübertragungseinrichtung mit
einem am proximalen Ende der Auslenkbewegungsübertragungseinrichtung angeordneten Steuerelement (1; 100) zur Bewerkstelligung einer Auslenkbewegung,
einem Basiskörper (3, 4; 300, 400), an dem das Steuerelement (1; 100) zur Bewerkstelligung einer Auslenkbewegung so angeordnet ist, dass eine Schwenkbewegung des Steuerelements (1; 100) relativ zum Basiskörper (3, 4; 300, 400) ausführbar ist,
einem länglichen Übertragungsführungskörper (5; 500), und
einem am distalen Ende der Auslenkbewegungsübertragungseinrichtung angeordneten auszulenkenden biegbaren Körper (6; 600),
wobei das Steuerelement (1; 100) einen Innenkanal (16; 160) aufweist, durch den der auszulenkende biegbare Körper (6; 600) hindurchführbar ist,
die Auslenkbewegungsübertragungseinrichtung zumindest einen Bewegungsübertragungsdrahtkörper (2, 2a, 2b) aufweist, dessen proximales Ende am Außenumfang des Schwenkabschnittes (11; 11') des Steuerelements (1; 100) angelenkt ist,
der Bewegungsübertragungsdrahtkörper (2, 2a, 2b) durch den länglichen Übertragungsführungskörper (5; 500) entlang seiner Längsrichtung der Bewegungsübertragungsdrahtkörper (2, 2a, 2b) geführt ist,
das distale Ende des Bewegungsübertragungsdrahtkörpers (2, 2a, 2b) am distalen Ende des auszulenkenden biegbaren Körpers (6; 600) angebracht ist,
wobei im Basiskörper (3, 4; 300, 400) ein Drahtkörperführungsring (7) zur Führung des Bewegungsübertragungsdrahtkörpers (2, 2a, 2b) eingesetzt ist, der relativ zum Basiskörper (3, 4; 300, 400) verschiebbar ist.

2. Auslenkbewegungsübertragungseinrichtung gemäß Anspruch 1,
wobei an einer Seite des Basiskörpers (3, 4; 300, 400) der längliche Übertragungsführungskörper (5; 500) angeordnet ist, und
der Basiskörper (3, 4; 300, 400) ebenfalls einen Innenkanal (35; 350) aufweist, durch den der auszulenkende biegbare Körper (6; 600) hindurchführbar ist.

3. Auslenkbewegungsübertragungseinrichtung gemäß Anspruch 2,
wobei der Innenkanal (16; 160) konzentrisch im Steuerelement (1; 100) angeordnet ist, und/oder
der Innenkanal (35; 350) konzentrisch im Basiskörper (3, 4; 300, 400) angeordnet ist;

4. Auslenkbewegungsübertragungseinrichtung gemäß einem der Ansprüche 2 oder 3, wobei
zumindest ein Abschnitt des länglichen Übertragungsführungskörpers (5; 500) durch den Innenkanal (16; 160) des Steuerelements (1; 100) und/oder den Innenkanal (35; 350) des Basiskörpers (3, 4; 300, 400) hindurchführbar ist.

5. Auslenkbewegungsübertragungseinrichtung gemäß einem der Ansprüche 1 bis 4, wobei
das Steuerelement (1; 100) einen Schwenkabschnitt (11; 110) besitzt, der an einem Kopfabschnitt (31; 310) des Basiskörpers (3, 4; 300, 400) abgestützt ist und zur Bewerkstelligung einer Auslenkbewegung relativ zum Kopfabschnitt (31; 31') des Basiskörpers (3, 4; 300, 400) schwenkbar ist, und
der Schwenkabschnitt (11; 110) des Steuerelements (1; 100) eine Ausgangsöffnung des Innenkanals (16; 160) des Steuerelement (100) besitzt und
der Kopfabschnitt (31; 310) des Basiskörpers (3, 4; 300, 400) eine Eingangsöffnung des Innenkanals (35; 350) des Basiskörpers (3, 4; 300, 400) besitzt.

6. Auslenkbewegungsübertragungseinrichtung gemäß Anspruch 5,
wobei das Steuerelement (100) als ein Betätigungshebel aufgebaut ist, der am Schwenkabschnitt (110) eine zum Basiskörper (300) weisende Fußfläche (110A) besitzt,
wobei der Kopfabschnitt (310) des Basiselements (300) eine zum Steuerelement (100) weisende Stirnfläche (310A) besitzt,
wobei zumindest die Fußfläche (110A) des Schwenkabschnitts (110) des Steuerelements (100) und/oder die Stirnfläche (310A) des Kopfabschnittes (310) des Basiselements (300) so gewölbt ist, dass die Fußfläche (110A) auf der Stirnfläche (310A) abrollbar ist,
wobei der Schwenkabschnitt (110) des Steuerelements (100) an seiner Außenseite den Anlenkpunkt des Bewegungsübertragungsdrahtkörpers (2, 2a, 2b) hat und mit seiner Fußfläche (110A) auf der Stirnfläche (310A) des Kopfabschnittes (310) des Basiselements (300) schwenkbar sitzt,
wobei die Fußfläche (110A) des Schwenkabschnitts (110) die Ausgangsöffnung des Innenkanals (160) des Steuerelement (100) aufweist und die Stirnfläche (310A) des Kopfabschnittes (310) die Eingangsöffnung des Innenkanals (350) des Basiskörpers (300, 400) aufweist.

7. Auslenkbewegungsübertragungseinrichtung gemäß Anspruch 5,
wobei das Steuerelement (1) als ein Betätigungshebel aufgebaut ist, der am Schwenkabschnitt (11) in einen Hohlkugelabschnitt übergeht, der zumindest als Kugelringabschnitt ausgebildet ist,
wobei der Kopfabschnitt (31) des Basiselements (3) kugelartig ausgebildet ist,
wobei der Hohlkugelabschnitt (11) an seiner Außenseite den Anlenkpunkt (14) des Bewegungsübertragungsdrahtkörpers (2, 2a, 2b) hat und an seiner Innenkugelfläche auf dem kugelartigen Kopfabschnitt (31) gleitfähig sitzt,
wobei der Hohlkugelabschnitt (11) die Ausgangsöffnung des Innenkanals (16) des Steuerelement (100) aufweist und der kugelartige Kopfabschnitt (31) die Eingangsöffnung des Innenkanals (35) des Basiskörpers (3, 4) aufweist.

8. Auslenkbewegungsübertragungseinrichtung gemäß einem der Ansprüche 1 bis 7, wobei das Steuerelement (1; 100) arretierbar ist, um eine Auslenkstellung des Steuerelements (1; 100) zu arretieren.

9. Auslenkbewegungsübertragungseinrichtung gemäß einem der Ansprüche 1 bis 8, wobei ein, zwei, drei, vier oder mehr Bewegungsübertragungsdrahtkörper (2, 2a, 2b) vorgesehen sind, von denen die ersten Enden gleichmäßig zueinander beabstandet am Steuerelement (1) angelenkt sind und die entgegengesetzten zweiten Enden am distalen Endabschnitt des auszulenkenden Körpers in entsprechender Weise gleichmäßig zueinander beabstandet befestigt sind.

10. Endoskopbiegesteuerung mit einer Auslenkbewegungsübertragungseinrichtung gemäß einem der Ansprüche 1 bis 9, wobei der Übertragungsführungskörper (5) ein Katheterschlauch ist, der auszulenkende Körper (6) ein Biegeabschnitt ist und der Biegeabschnitt und ein Abschnitt des Katheterschlauches durch das Steuerelement (1; 100) hindurchführbar ist.

11. Endoskop mit einer Endoskopbiegesteuerung gemäß Anspruch 10.

## Claims

1. Deflection movement transmission device having a control element (1; 100) for bringing about a deflection movement, which control element (1; 100) is arranged at the proximal end of the deflection movement transmission device,
a base body (3, 4; 300, 400), on which the control element (1; 100) for bringing about a deflection movement is arranged in such a way that a pivoting movement of the control element (1; 100) can be carried out relative to the base body (3, 4; 300, 400),
an elongate transmission guide body (5; 500), and
a flexible body (6; 600) to be deflected which is arranged at the distal end of the deflection movement transmission device,
the control element (1; 100) having an inner channel (16; 160), through which the flexible body (6; 600) to be deflected can be guided,
the deflection movement transmission device having at least one movement transmission wire body (2, 2a, 2b), the proximal end of which is articulated on the outer circumference of the pivoting section (11; 11') of the control element (1; 100),
the movement transmission wire body (2, 2a, 2b) being guided by way of the elongate transmission guide body (5; 500) along the longitudinal direction of the movement transmission wire body (2, 2a, 2b),
the distal end of the movement transmission wire body (2, 2a, 2b) being attached to the distal end of the flexible body (6; 600) to be deflected,
a wire body guide ring (7) for guiding the movement transmission wire body (2, 2a, 2b) being inserted in the base body (3, 4; 300, 400), which movement transmission wire body (2, 2a, 2b) can be displaced relative to the base body (3, 4; 300, 400).

2. Deflection movement transmission device according to Claim 1,
the elongate transmission guide body (5; 500) being arranged on one side of the base body (3, 4; 300, 400), and
the base body (3, 4; 300, 400) likewise having an inner channel (35; 350), through which the flexible body (6; 600) to be deflected can be guided.

3. Deflection movement transmission device according to Claim 2,
the inner channel (16; 160) being arranged concentrically in the control element (1; 100), and/or the inner channel (35; 350) being arranged concentrically in the base body (3, 4; 300, 400).

4. Deflection movement transmission device according to either of Claims 2 and 3,
it being possible for at least one section of the elongate transmission guide body (5; 500) to be guided through the inner channel (16; 160) of the control element (1; 100) and/or the inner channel (35; 350) of the base body (3, 4; 300, 400).

5. Deflection movement transmission device according to one of Claims 1 to 4,
the control element (1; 100) having a pivoting section (11; 110) which is supported on a head section (31; 310) of the base body (3, 4; 300, 400) and can be pivoted in order to bring about a deflection movement relative to the head section (31; 31') of the base body (3, 4; 300, 400), and
the pivoting section (11; 110) of the control element (1; 100) having an outlet opening of the inner channel (16; 160) of the control element (100), and
the head section (31; 310) of the base body (3, 4; 300, 400) having an inlet opening of the inner channel (35; 350) of the base body (3, 4; 300, 400).

6. Deflection movement transmission device according to Claim 5,
the control element (100) being constructed as an actuating lever which, on the pivoting section (110), has a bottom face (110A) which points towards the base body (300),
the head section (310) of the base element (300) having an end face (310A) which points towards the control element (100),
at least the bottom face (110A) of the pivoting section (110) of the control element (100) and/or the end face (310A) of the head section (310) of the base element (300) being curved in such a way that the bottom face (110A) can roll on the end face (310A),
the pivoting section (110) of the control element (100) having, on its outer side, the articulation point of the movement transmission wire body (2, 2a, 2b), and being seated with its bottom face (110A) pivotably on the end face (310A) of the head section (310) of the base element (300),
the bottom face (110A) of the pivoting section (110) having the outlet opening of the inner channel (160) of the control element (100), and the end face (310A) of the head section (310) having the inlet opening of the inner channel (350) of the base body (300, 400).

7. Deflection movement transmission device according to Claim 5,
the control element (1) being configured as an actuating lever which merges at the pivoting section (11) into a hollow sphere section which is configured at least as a sphere ring section,
the head section (31) of the base element (3) being of spherical configuration,
the hollow sphere section (11) having, on its outer side, the articulation point (14) of the movement transmission wire body (2, 2a, 2b), and being seated in a slidable manner on its inner sphere face on the spherical head section (31),
the hollow sphere section (11) having the outlet opening of the inner channel (16) of the control element (100), and the spherical head section (31) having the inlet opening of the inner channel (35) of the base body (3, 4).

8. Deflection movement transmission device according to one of Claims 1 to 7, it being possible for the control element (1; 100) to be locked, in order to lock a deflected position of the control element (1; 100).

9. Deflection movement transmission device according to one of Claims 1 to 8, one, two, three, four or more movement transmission wire bodies (2, 2a, 2b) being provided, of which the first ends are articulated on the control element (1) in a manner which is spaced apart homogeneously from one another, and the opposite second ends of which are fastened to the distal end section of the body to be deflected in a corresponding way in a manner which is spaced apart homogeneously from one another.

10. Endoscope bending controller having a deflection movement transmission device according to one of Claims 1 to 9, the transmission guide body (5) being a catheter tube, the body (6) to be deflected being a bending section, and it being possible for the bending section and a section of the catheter tube to be guided through the control element (1; 100).

11. Endoscope having an endoscope bending controller according to Claim 10.

## Revendications

1. Dispositif de transmission d'un mouvement de déviation, comprenant :
un élément de commande (1 ; 100) disposé à l'extrémité proximale du dispositif de transmission de mouvement de déviation pour produire un mouvement de déviation,
un corps de base (3, 4 ; 300, 400), au niveau duquel l'élément de commande (1 ; 100) destiné à produire un mouvement de déviation est disposé de telle sorte qu'un mouvement de pivotement de l'élément de commande (1 ; 100) par rapport au corps de base (3, 4 ; 300, 400) puisse être réalisé,
un corps de guidage de transmission allongé (5 ; 500), et
un corps flexible (6 ; 600) à dévier, disposé au niveau de l'extrémité distale du dispositif de transmission de mouvement de déviation,
l'élément de commande (1 ; 100) présentant un canal interne (16 ; 160), à travers lequel peut être guidé le corps flexible à dévier (6 ; 600),
le dispositif de transmission de mouvement de déviation présentant au moins un corps de fil de transmission de mouvement (2, 2a, 2b), dont l'extrémité proximale est articulée à la périphérie extérieure de la portion de pivotement (11 ; 11') de l'élément de commande (1 ; 100),
le corps de fil de transmission de mouvement (2, 2a, 2b) étant guidé par le corps de guidage de transmission allongé (5 ; 500) le long de sa direction longitudinale de corps de fil de transmission de mouvement (2, 2a, 2b),
l'extrémité distale du corps de fil de transmission de mouvement (2, 2a, 2b) étant montée à l'extrémité distale du corps flexible à dévier (6 ; 600),
une bague de guidage de corps de fil (7) pour guider le corps de fil de transmission de mouvement (2, 2a, 2b) étant insérée dans le corps de base (3, 4 ; 300, 400), laquelle peut être déplacée par rapport au corps de base (3, 4 ; 300, 400) .

2. Dispositif de transmission d'un mouvement de déviation selon la revendication 1, dans lequel
le corps de guidage de transmission allongé (5 ; 500) est disposé au niveau d'un côté du corps de base (3, 4 ; 300, 400), et
le corps de base (3, 4 ; 300, 400) présente également un canal interne (35 ; 350) à travers lequel peut être guidé le corps flexible à dévier (6 ; 600).

3. Dispositif de transmission d'un mouvement de déviation selon la revendication 2, dans lequel le canal interne (16 ; 160) est disposé concentriquement dans l'élément de commande (1 ; 100), et/ou
le canal interne (35 ; 350) est disposé concentriquement dans le corps de base (3, 4 ; 300, 400).

4. Dispositif de transmission d'un mouvement de déviation selon l'une quelconque des revendications 2 ou 3, dans lequel
au moins une portion du corps de guidage de transmission allongé (5 ; 500) peut être guidée à travers le canal interne (16 ; 160) de l'élément de commande (1 ; 100) et/ou à travers le canal interne (35 ; 350) du corps de base (3, 4 ; 300, 400) .

5. Dispositif de transmission d'un mouvement de déviation selon l'une quelconque des revendications 1 à 4, dans lequel
l'élément de commande (1 ; 100) possède une portion pivotante (11 ; 110) qui est supportée au niveau d'une portion de tête (31 ; 310) du corps de base (3, 4 ; 300, 400) et qui peut pivoter par rapport à la portion de tête (31 ; 31') du corps de base (3, 4 ; 300, 400) pour provoquer un mouvement de déviation, et
la portion pivotante (11 ; 110) de l'élément de commande (1 ; 100) possède une ouverture de sortie du canal interne (16 ; 160) de l'élément de commande (100) et
la portion de tête (31 ; 310) du corps de base (3, 4 ; 300, 400) possède une ouverture d'entrée du canal interne (35 ; 350) du corps de base (3, 4 ; 300, 400).

6. Dispositif de transmission d'un mouvement de déviation selon la revendication 5, dans lequel
l'élément de commande (100) est réalisé sous forme de levier d'actionnement qui possède, au niveau de la portion pivotante (110), une surface de pied (110A) orientée vers le corps de base (300),
la portion de tête (310) de l'élément de base (300) possède une surface frontale (310A) tournée vers l'élément de commande (100),
au moins la surface de pied (110A) de la portion pivotante (110) de l'élément de commande (100) et/ou la surface frontale (310A) de la portion de tête (310) de l'élément de base (300) est cintrée de telle sorte que la surface de pied (110A) puisse rouler sur la surface frontale (310A),
la portion pivotante (110) de l'élément de commande (100) présente, au niveau de son côté extérieur, le point d'articulation du corps de fil de transmission de mouvement (2, 2a, 2b), et repose de manière pivotante avec sa surface de pied (110A) sur la surface frontale (310A) de la portion de tête (310) de l'élément de base (300),
la surface de pied (110A) de la portion pivotante (110) présente l'ouverture de sortie du canal interne (160) de l'élément de commande (100) et la surface frontale (310A) de la portion de tête (310) présente l'ouverture d'entrée du canal interne (350) du corps de base (300, 400).

7. Dispositif de transmission d'un mouvement de déviation selon la revendication 5, dans lequel
l'élément de commande (1) est réalisé sous forme de levier d'actionnement qui se prolonge au niveau de la portion pivotante (11) par une portion sphérique creuse qui est réalisée au moins sous forme de portion de bague sphérique,
la portion de tête (31) de l'élément de base (3) est réalisée sous forme sphérique,
la portion sphérique creuse (11) présente, au niveau de son côté extérieur, le point d'articulation (14) du corps de fil de transmission de mouvement (2, 2a, 2b) et repose de manière coulissante au niveau de sa surface sphérique interne sur la portion de tête sphérique (31),
la portion sphérique creuse (11) présente l'ouverture de sortie du canal interne (16) de l'élément de commande (100) et la portion de tête sphérique (31) présente l'ouverture d'entrée du canal interne (35) du corps de base (3, 4).

8. Dispositif de transmission d'un mouvement de déviation selon l'une quelconque des revendications 1 à 7, dans lequel l'élément de commande (1 ; 100) peut être bloqué afin de bloquer une position de déviation de l'élément de commande (1 ; 100).

9. Dispositif de transmission d'un mouvement de déviation selon l'une quelconque des revendications 1 à 8, dans lequel un, deux, trois, quatre ou plus de quatre corps de fil de transmission de mouvement (2, 2a, 2b) sont prévus, dont les premières extrémités sont articulées à l'élément de commande (1) de manière équidistante et les deuxièmes extrémités opposées sont fixées de manière équidistante correspondante à la portion d'extrémité distale du corps à dévier.

10. Commande de la courbure d'un endoscope comprenant un dispositif de transmission d'un mouvement de déviation selon l'une quelconque des revendications 1 à 9, dans laquelle le corps de guidage de transmission (5) est un tube de cathéter, le corps à dévier (6) est une portion courbe et la portion courbe et une portion du tube de cathéter peuvent être guidées à travers l'élément de commande (1 ; 100).

11. Endoscope comprenant une commande de la courbure d'un endoscope selon la revendication 10.
